# EUROPEAN PATENT APPLICATION

(11) **EP 4 327 826 A1**
(43) Date of publication of application: **28.02.2024**
(21) Application number: 22791733.3
(22) Date of filing: 19.04.2022
(51) Int. Cl.: A61K 45/06, A61K 31/20, A61K 31/423, A61P 3/06, A61P 9/00, A61P 9/10, A61P 43/00

(54) **COMBINATION MEDICINE FOR PREVENTING/TREATING DYSLIPIDEMIA OR CARDIOVASCULAR DISEASE**

(30) Priority: 20.04.2021 JP 2021071269
(71) Applicant: KOWA COMPANY, LTD., Naka-ku Nagoya-shi Aichi 460-8625 (JP)
(72) Inventor: INOUE, Noriyuki, Higashimurayama-shi, Tokyo 189-0022 (JP); SAITO, Kenji, Higashimurayama-shi, Tokyo 189-0022 (JP); TAKIZAWA, Toshiaki, Higashimurayama-shi, Tokyo 189-0022 (JP)
(74) Representative: Kraus & Lederer PartGmbB
(86) International application number: PCT/JP2022/018192
(87) International publication number: WO 2022/224962

(57) **Abstract**

The present invention addresses the problem of providing a pharmaceutical composition/drug combination which makes it possible to prevent and/or treat dyslipidemia such as hypercholesterolemia and hypertriglyceridemia, or cardiovascular diseases such as arteriosclerosis and cardiovascular events. The present invention provides a combination of a peroxisome proliferator-activated receptor (PPAR) α agonist and an ATP citrate lyase (ACL) inhibitor for preventing and/or treating dyslipidemia such as hypercholesterolemia and hypertriglyceridemia, or cardiovascular diseases such as arteriosclerosis and cardiovascular events.

## Description

### Technical Field

The present invention relates to, for example, a combination drug or a combination of drugs for preventing and/or treating dyslipidemia such as atherosclerosis or hypercholesterolemia, or cardiovascular diseases such as arteriosclerosis and cardiovascular events.

### Background Art

Atherosclerotic diseases cause ischemic diseases such as myocardial infarction and cerebrovascular disorders such as cerebral infarction that develop on the basis of arteriosclerosis, and account for the second largest cause of death next to cancer. Dyslipidemia, that is, hyper LDL cholesterolemia (LDL-C), hypertriglyceridemia, hypo HDL cholesterolemia (HDL-C), and hyper non-HDL-C and the like, against the background of diversified lifestyles and rapidly aging population, is on the rise, and the dyslipidemia state is a risk factor of arteriosclerosis. In particular, hyper LDL cholesterolemia, hypo HDL cholesterolemia, and hypertriglyceridemia have been epidemiologically proven to be risk factors of arteriosclerotic diseases, and "Guidelines for Prevention of Atherosclerotic Cardiovascular Diseases, 2017 edition" and "Medical Guide for Prevention of Atherosclerotic Diseases, 2018 edition" by the Japan Atherosclerosis Society also show the importance of the management of the dyslipidemia.

Dyslipidemia, particularly hypercholesterolemia, has already become a disease area with considerably high medical satisfaction due to the advent of statins. However, from the results of many large-scale clinical trials, it has been found that further lowering of LDL-C in the blood leads to prevention of coronary artery disease (the lower, the better), and more strict lipid control is required. There are many patients who cannot reach a target LDL-C value in the blood only with a statin, and combination use of multiple drugs is also required.

Not only dyslipidemia but also hypertension, diabetes, chronic kidney disease, obesity, and metabolic syndrome are all suggested to be associated with coronary artery disease (CAD) as risk factors. It has been reported that a high level of triglyceride (TG) is associated with the risk of coronary artery disease, that diabetes is a disease state at a high risk of arteriosclerotic disease, and that metabolic syndrome represented by obesity is also a lesion at a high risk of onset of cardiovascular disease (Non-Patent Document 1). Nonalcoholic fatty liver disease (NAFLD)/nonalcoholic steatohepatitis (NASH) is one phenotype in the liver with metabolic syndrome and has been frequently reported to be a risk factor for coronary artery disease and diabetes and the like (Non-Patent Document 2).

Peroxisome Proliferator-Activated Receptor (PPAR) is one of receptors belonging to a nuclear receptor family. The existence of three subtypes (α, γ, and δ) has been known for this receptor (Non-Patent Document 3). Among them, PPARα is mainly expressed in the liver, and when PPARα is activated, production of apo C-III is suppressed, followed by activation of lipoprotein lipase (LPL). As a result, fat is decomposed. As PPARα agonists, unsaturated fatty acids, and fibrate-based drugs such as fenofibrate, bezafibrate, and gemfibrozil have been known (Non-Patent Document 4). In recent years, a compound having a stronger and more selective PPARα activating effect than that of a conventional fibrate-based drug has been reported (Patent Document 1).

ATP citrate lyase (hereinafter referred to as "ACL") is an enzyme that is located most upstream of a lipid synthesis system, and produces acetyl CoA, which is a material for lipid synthesis, from citric acid, which is a glycolytic intermediate, in cells. ACL has been reported to be abnormally expressed in many cancers, cardiovascular diseases and metabolic disorders, and the Mendelia randomization of large-scale human cohort validated that ACL can be a promising therapeutic target for LDL-C reduction and protection of atherosclerosis (Non-Patent Document 5). As ACL inhibitors, drugs such as 8-hydroxy-2,2,14,14-tetramethylpentadecanedioic acid (bempedoic acid, Patent Document 2) and SB-204990 (Non-Patent Document 6) have been known.

### Citation List

### Patent Document

Patent Document 1: WO 2005/023777 A1
Patent Document 2: WO 2004/067489 A1

### Non-Patent Document

Non-Patent Document 1: Guidelines for Prevention of Atherosclerotic Cardiovascular Diseases (2017 edition; published by Japan Atherosclerosis Society)
Non-Patent Document 2: Clinical Practice Guidelines for NAFLD/NASH 2020 (edited by The Japanese Society of Gastroenterology and The Japan Society of Hepatology)
Non-Patent Document 3: J. Lipid Research, 37, 907-925 (1996)
Non-Patent Document 4: Trends in Endocrinology and Metabolism, 15 (7), 324-330 (2004)
Non-Patent Document 5: Progress in Lipid Research 77 (2020) 101006
Non-Patent Document 6: Biochem. J., 334 (1) 113-119 (1998)

### Summary of the Invention

### Problems to be Solved by the Invention

An object of the present invention is to provide a combination pharmaceutical composition and a combination of drugs for preventing and/or treating dyslipidemia such as hypercholesterolemia and hypertriglyceridemia, or cardiovascular diseases such as arteriosclerosis and cardiovascular events.

### Means for Solving the Problems

In view of such circumstances, the present inventors have extensively conducted studies, and resultantly found that (R)-2-[3-[[N-(benzoxazole-2-yl)-N-3-(4-methoxyphenoxy)propyl]aminomethyl]phenoxy]butyric acid (Example 85 of Patent Document 1: hereinafter, may be referred to as a compound A), which has been reported as a selective PPARα activator, and bempedoic acid (Compound 339 in Patent Document 2: hereinafter, may be referred to as a compound B) are used in combination to exhibit a cholesterol lowering action in the liver and a TG lowering action in the blood, thereby completing the present invention.

That is, the present invention relates to a composition and a kit, and the like characterized by a combination of a PPARα agonist and an ACL inhibitor. More specifically, the present invention relates to the following [1] to [39].
[1] A prophylactic and/or therapeutic agent for dyslipidemia or cardiovascular disease, comprising a combination of a PPARα agonist and an ACL inhibitor.
[2] The prophylactic and/or therapeutic agent according to above [1], wherein the dyslipidemia is hypercholesterolemia or hypertriglyceridemia.
[3] The prophylactic and/or therapeutic agent according to above [1] or [2], wherein the cardiovascular disease is arteriosclerosis or a cardiovascular event.
[4] The prophylactic and/or therapeutic agent according to any one of above [1] to [3], wherein the PPARα agonist is (R)-2-[3-[[N-(benzoxazole-2-yl)-N-3-(4-methoxyphenoxy)propyl]aminomethyl]phenoxy]butyric acid, or a salt, or a solvate, or a solvate of the salt thereof.
[5] The prophylactic and/or therapeutic agent according to any one of above [1] to [4], wherein the ACL inhibitor is 8-hydroxy-2,2,14,14-tetramethylpentadecanedioic acid, or a salt, or a solvate, or a solvate of the salt thereof.
[6] The prophylactic and/or therapeutic agent according to any one of above [1] to [5], wherein the prophylactic and/or therapeutic agent is a combination drug.
[7] The prophylactic and/or therapeutic agent according to any one of above [1] to [5], wherein the prophylactic and/or therapeutic agent is a kit.
[8]A medicament for use in prevention and/or treatment of dyslipidemia or cardiovascular disease, comprising a combination of a PPARα agonist and an ACL inhibitor.
[9] The medicament for use according to above [8], wherein the dyslipidemia is hypercholesterolemia or hypertriglyceridemia.
[10] The medicament for use according to above [8] or [9], wherein the cardiovascular disease is arteriosclerosis or a cardiovascular event.
[11] The medicament for use according to any one of above [8] to [10], wherein the PPARα agonist is (R)-2-[3-[[N-(benzoxazole-2-yl)-N-3-(4-methoxyphenoxy)propyl]aminomethyl]phenoxy]butyric acid, or a salt, or a solvate, or a solvate of the salt thereof.
[12] The medicament for use according to any one of above [8] to [11], wherein the ACL inhibitor is 8-hydroxy-2,2,14,14-tetramethylpentadecanedioic acid, or a salt, or a solvate, or a solvate of the salt thereof.
[13] The medicament for use according to any one of above [8] to [12], wherein the medicament is a combination drug.
[14] The medicament for use according to any one of above [8] to [13], wherein the medicament is a kit.
[15] A pharmaceutical composition for preventing and/or treating dyslipidemia or cardiovascular disease, comprising a PPARα agonist and an ACL inhibitor.
[16] The pharmaceutical composition according to above [15], wherein the dyslipidemia is hypercholesterolemia or hypertriglyceridemia.
[17] The pharmaceutical composition according to above [15] or [16], wherein the cardiovascular disease is arteriosclerosis or a cardiovascular event.
[18] The pharmaceutical composition according to any one of above [15] to [17], wherein the PPARα agonist is (R)-2-[3-[[N-(benzoxazole-2-yl)-N-3-(4-methoxyphenoxy)propyl]aminomethyl]phenoxy]butyric acid, or a salt, or a solvate, or a solvate of the salt thereof.
[19] The pharmaceutical composition according to any one of above [15] to [18], wherein the ACL inhibitor is 8-hydroxy-2,2,14,14-tetramethylpentadecanedioic acid, or a salt, or a solvate, or a solvate of the salt thereof.
[20] The pharmaceutical composition according to any one of above [15] to [19], further containing a pharmaceutically acceptable carrier.
[21] A method for preventing and/or treating dyslipidemia or cardiovascular disease, comprising: a step of administering an effective amount of a PPARα agonist and an effective amount of an ACL inhibitor to a subject in need of treatment.
[22] The method according to above [21], wherein the dyslipidemia is hypercholesterolemia or hypertriglyceridemia.
[23] The method according to above [21] or [22], wherein the cardiovascular disease is arteriosclerosis or a cardiovascular event.
[24] The method according to any one of above [21] to [23], wherein the PPARα agonist is (R)-2-[3-[[N-(benzoxazole-2-yl)-N-3-(4-methoxyphenoxy)propyl]aminomethyl]phenoxy]butyric acid, or a salt, or a solvate, or a solvate of the salt thereof.
[25] The method according to any one of above [21] to [24], wherein the ACL inhibitor is 8-hydroxy-2,2,14,14-tetramethylpentadecanedioic acid, or a salt, or a solvate, or a solvate of the salt thereof.
[26] Use of a PPARα agonist and an ACL inhibitor for the production of a prophylactic and/or therapeutic agent for dyslipidemia or cardiovascular disease.
[27] The use according to above [26], wherein the dyslipidemia is hypercholesterolemia or hypertriglyceridemia.
[28] The use according to above [26] or [27], wherein the cardiovascular disease is arteriosclerosis or a cardiovascular event.
[29] The use according to any one of above [26] to [28], wherein the PPARα agonist is (R)-2-[3-[[N-(benzoxazole-2-yl)-N-3-(4-methoxyphenoxy)propyl]aminomethyl]phenoxy]butyric acid, or a salt, or a solvate, or a solvate of the salt thereof.
[30] The use according to any one of above [26] to [29], wherein the ACL inhibitor is 8-hydroxy-2,2,14,14-tetramethylpentadecanedioic acid, or a salt, or a solvate, or a solvate of the salt thereof.
[31] The use according to any one of above [26] to [30], wherein the prophylactic and/or therapeutic agent is a combination drug.
[32] The use according to any one of above [26] to [30], wherein the prophylactic and/or therapeutic agent is a kit.
[33] A combination of a PPARα agonist and an ACL inhibitor for preventing and/or treating dyslipidemia or cardiovascular disease.
[34] The combination according to above [33], wherein the dyslipidemia is hypercholesterolemia or hypertriglyceridemia.
[35] The combination according to above [33] or [34], wherein the cardiovascular disease is arteriosclerosis or a cardiovascular event.
[36] The combination according to any one of above [33] to [35], wherein the PPARα agonist is (R)-2-[3-[[N-(benzoxazole-2-yl)-N-3-(4-methoxyphenoxy)propyl]aminomethyl]phenoxy]butyric acid, or a salt, or a solvate, or a solvate of the salt thereof.
[37] The combination according to any one of above [33] to [36], wherein the ACL inhibitor is 8-hydroxy-2,2,14,14-tetramethylpentadecanedioic acid, or a salt, or a solvate, or a solvate of the salt thereof.
[38] The combination according to any one of above [33] to [37], wherein the prophylactic and/or therapeutic agent is a combination drug.
[39] The combination according to any one of above [33] to [37], wherein the prophylactic and/or therapeutic agent is a kit.

### Effects of the Invention

The therapeutic agent, medicament, pharmaceutical composition, therapeutic method, use or combination of the present invention have an excellent total cholesterol lowering action in the liver and an excellent TG lowering action in the blood, and can provide prevention and/or treatment for dyslipidemia such as hypercholesterolemia and hypertriglyceridemia, or cardiovascular diseases such as arteriosclerosis and cardiovascular events.

### Brief Description of Drawings

FIG. 1 is a diagram showing a TG index (%) when a compound A (0.3 mg/kg) and a compound B (30 mg/kg) are administered alone or in combination to a Wistar rat once a day for 2 weeks. Ctrl represents control of solvent administration.
FIG. 2 is a diagram showing the expression level (relative expression level of mRNA) of PPARα gene in the liver when a compound A (0.3 mg/kg) and a compound B (30 mg/kg) are administered alone or in combination to a Wistar rat once a day for 2 weeks. Ctrl represents control of solvent administration.
FIG. 3 is a diagram showing a total cholesterol value (mg/g) in the liver of each of db/db mice when a compound A (0.3 mg/kg) and a compound B (10 mg/kg) are administered alone or in combination once a day for 2 weeks. Ctrl represents control of solvent administration.

### Description of Embodiments

In the present invention, a PPARα agonist means a generic term of compounds that activate a PPARα-type receptor involved in fat oxidation and the like in a peroxisome proliferator-activated receptor (PPAR) that is one of nuclear receptors. Specific examples thereof include fibrates such as fenofibrate, clofibrate, bezafibrate, clinofibrate, ciprofibrate, etofibrate, and gemfibrozil, and WY-14643 (pirinixic acid), GW-7647, and pemafibrate.

(R)-2-[3-[[N-(benzoxazole-2-yl)-N-3-(4-methoxyphenoxy)propyl]aminomethyl]phenoxy]butyric acid (compound A) used in Examples of the present invention is also known as the common name "pemafibrate", and the compound A is described by the chemical structural formula as follows.

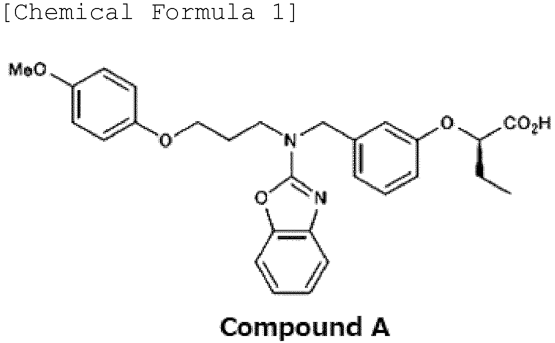

The compound A can be produced, for example, in accordance with a method described in WO 2005/023777 A1 (Patent Document 1) or the like. The compound A can also be produced in accordance with a method described in the literature. Furthermore, in the present invention, a salt or solvate of the compound A can also be used. The salt and the solvate can be produced by a conventional method.

The salt of the compound A is not particularly limited as long as being pharmaceutically acceptable. However, examples thereof include an alkali metal salt such as a sodium salt or a potassium salt; an alkaline earth metal salt such as a calcium salt or a magnesium salt; an organic base salt such as an ammonium salt or a trialkylamine salt; a mineral acid salt such as a hydrochloride or a sulfate; and an organic acid salt such as an acetate.

Examples of the solvate of the compound A or the salt of the compound A include a hydrate and an alcohol solvate (for example, ethanol solvate).

In the present invention, ATP citrate lyase (ACL) is an enzyme that is located most upstream of a lipid synthesis system, and produces acetyl CoA, which is a material for lipid synthesis, from citric acid, which is a glycolytic intermediate, in cells. An ACL inhibitor is a generic term for substances that inhibit the enzyme ACL. Examples of the ACL inhibitor include 8-hydroxy-2,2,14,14-tetramethylpentadecanedioic acid (bempedoic acid, compound B), SB-204990, curcumin, resveratrol, cinnamon polyphenol, hydroxycitric acid, baker's yeast glucan, Medica 16, 3-thiadicarboxylic acid, and 2-hydroxy-N-arylbenzenesulfonamide.

The 8-hydroxy-2,2,14,14-tetramethylpentadecanedioic acid (compound B) used in Examples of the present invention is also otherwise known as bempedoic acid, and the compound B is described in the chemical structural formula as follows.

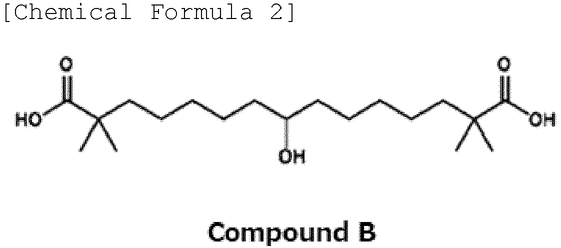

The compound B can be produced, for example, in accordance with a method described in WO 2004/067489 A1 (Patent Document 2) or the like. The compound B can also be produced in accordance with a method described in the literature. Furthermore, in the present invention, a salt or solvate of the compound B can also be used. The salt and the solvate can be produced by a conventional method.

The salt of the compound B is not particularly limited as long as being pharmaceutically acceptable. However, examples thereof include an alkali metal salt such as a sodium salt or a potassium salt; an alkaline earth metal salt such as a calcium salt or a magnesium salt; an organic base salt such as an ammonium salt or a trialkylamine salt; a mineral acid salt such as a hydrochloride or a sulfate; and an organic acid salt such as an acetate.

Examples of the solvate of the compound B or the salt of the compound B include a hydrate and an alcohol solvate (for example, ethanol solvate).

In the present specification, the term "dyslipidemia" means a case where any one of a total triglyceride (TG) level, a total cholesterol (TC) level, a VLDL cholesterol (VLDL-C) level, an LDL cholesterol (LDL-C) level, or an HDL cholesterol (HDL-C) level in the blood, or two or more kinds thereof deviate from a range of normal values. A case where the LDL cholesterol (LDL-C) level deviates from a range of normal values or a case where the total triglyceride (TG) level deviates from a range of normal values is a preferable treatment subject with dyslipidemia in the present invention. A disease requiring lowering of LDL cholesterol (LDL-C) in the present invention refers to a case where the LDL-C level in the blood is higher than a normal value, and a disease requiring lowering of total triglyceride (TG) in the present invention refers to a case where the TG level in the blood is higher than a normal value.

In the present invention, the term "cardiovascular disease" means a disease group in which the lumen of a blood vessel is narrowed mainly by arteriosclerosis, resulting in insufficient supply of oxygen-rich blood to the organ. Specifically, the cardiovascular disease includes the following three categories, that is, coronary artery disease, cerebral infarction, and peripheral arterial disease.

The term "combination drug" is a pharmaceutical product containing a plurality of active ingredients in a single formulation. In the present invention, examples of the combination drug include a tablet containing an effective amount of the PPARα agonist and an effective amount of the ACL inhibitor.

The term "kit" is a combination of pharmaceutical products for taking or administering a plurality of drugs simultaneously or separately at intervals. In the present invention, examples of the kit include a combination of a pharmaceutical product containing an effective amount of the PPARα agonist with a pharmaceutical product containing an effective amount of the ACL inhibitor.

The pharmaceutical composition of the present invention can be formed into a dosage form such as a tablet, a capsule, a granule, a powder, a lotion, an ointment, an injection, or a suppository singly or using another pharmaceutically acceptable carrier. These formulations can be produced by a known method. For example, when a formulation for oral administration is produced, the formulation can be produced by appropriately combining and formulating a dissolving agent such as gum tragacanth, gum arabic, a sucrose fatty acid ester, lecithin, olive oil, soybean oil, or PEG400; an excipient such as starch, mannitol, or lactose; a binder such as methyl cellulose, sodium carboxymethylcellulose, or hydroxypropylcellulose; a disintegrating agent such as crystalline cellulose or calcium carboxymethylcellulose; a lubricant such as talc or magnesium stearate; and a flow improver such as light anhydrous silicic acid.

As the use form of the pharmaceutical composition of the present invention, a) a compound A, or a salt, or a solvate, or a solvate of the salt thereof, and b) a compound B, or a salt, or a solvate, or a solvate of the salt thereof are combined. It is possible to use a form in which an effect for preventing and/or treating dyslipidemia such as hypercholesterolemia, hyper LDL-C cholesterolemia, or hyper TG cholesterolemia, and an effect for preventing and/or treating cardiovascular diseases such as arteriosclerosis and cardiovascular events are obtained by using synergistic effects for lowering LDL-C and lowering TG due to administration of the two drugs in addition to an effect by each of the drugs. However, the present invention is not limited to the use form thereof. The compound A, or a salt, or a solvate, or a solvate of the salt thereof, and the compound B, or a salt, or a solvate, or a solvate of the salt thereof may be administered simultaneously, or may be administered separately (for example, at different times during the same day) at an interval.

The compound A, or a salt, or a solvate, or a solvate of the salt thereof, and the compound B, or a salt, or a solvate, or a solvate of the salt thereof may be formulated into a single formulation, or the two drugs may be formulated separately to be used as a kit. That is, the pharmaceutical composition of the present invention may be a kit formed by combining a drug containing at least one selected from the compound A, a salt thereof, a solvate thereof, and a solvate of the salt thereof as an active ingredient, and a drug containing at least one selected from the compound B, a salt thereof, a solvate thereof, and a solvate of the salt thereof.

When the compound A, or a salt, or a solvate, or a solvate of the salt thereof, and the compound B, or a salt, or a solvate, or a solvate of the salt thereof are formulated separately, dosage forms of the two drugs may be the same as or different from each other. The numbers of administration for ingredients may be different from each other.

The compound A, or a salt, or a solvate, or a solvate of the salt thereof of the present invention is administered orally or parenterally. The dose of the pharmaceutical agent of the present invention varies depending on a patient's weight, age, sex, and symptoms, and the like. However, in a case of an adult, it is usually desirable to administer 0.001 to 1000 mg, preferably 0.01 to 100 mg, and particularly preferably 0.01 to 20 mg of the compound A per day in one to three parts. As for the compound B, or a salt, or a solvate, or a solvate of the salt thereof, it is desirable to administer 1 to 10000 mg, preferably 10 to 5000 mg, and particularly preferably 50 to 1000 mg of the compound B per day in one to three parts.

In the present invention, when the two drugs are administered as a single formulation, a blending ratio between the compound A, or a salt, or a solvate, or a solvate of the salt thereof, and the compound B, or a salt, or a solvate, or a solvate of the salt thereof can be appropriately selected in a range of an effective dose of each active ingredient, but in general, is preferably 1:1 to 1:10000, more preferably 1:5 to 1:4000, and particularly preferably 1:10 to 1:1000 in terms of the mass ratio of the compound B to the compound A. The range of the mass ratio of the dose per day when the compound A, or a salt, or a solvate, or a solvate of the salt thereof, and the compound B, or a salt, or a solvate, or a solvate of the salt thereof are formulated separately is also the same as described above.

### Examples

Hereinafter, the present invention will be described more specifically with reference to Examples, but the present invention is not limited by these Examples at all.

### Example 1 Effect of Combination Use of Compound A and Compound B on TG Index of Rat

### 1. Method

Male rats (7-week-old, Wistar [Crlj: WI], Charles River Laboratories Japan, Inc.) were used for an experiment. Blood was collected from the jugular vein under satiation, and the rats were divided into four groups (N=6) based on TG and TC in the plasma and body weights. For two weeks from the next day of the grouping, each of a solvent (0.5% carboxymethyl cellulose aqueous solution: CMC), a compound A (pemafibrate) alone, a compound B (bempedoic acid) alone, and a combination of pemafibrate and bempedoic acid was orally administered once per day. After four hours from the final administration of the drug, blood was collected under isoflurane anesthesia to obtain plasma. Parameters in the blood were measured using an automated analyzer (Labospect 003, Hitachi High-Technologies Corporation). A lipid content in the liver was measured using the method described in J. Biol. Chem. 226 (1), 497-509 (1957) (FOLCH method). A TG index (%) was expressed as a ratio of a product of a blood TG concentration and a TG content in the liver of a sample to that of Control.

In the present Example, the compound A was prepared and used according to the method described in Patent Document 1. In accordance with the method described in Patent Document 2, bempedoic acid manufactured by MedChemExpress was prepared and used as the compound B.

### 2. Group Configuration

| | |
|---|---|
| Group 1: | Control (Solvent Only) |
| Group 2: | 0.3 mg/kg of Compound A |
| Group 3: | 30 mg/kg of Compound B |
| Group 4: | 0.3 mg/kg of Compound A and 30 mg/kg of Compound B |

### 3. Statistical Analysis and Data Processing Method

The results were presented by an average value ± standard error. Comparison between the control group and each of the drug administration groups was performed by a multiple comparison test of Dunnett, and a risk rate of less than 5% was determined to have a significant difference.

### 4. Results

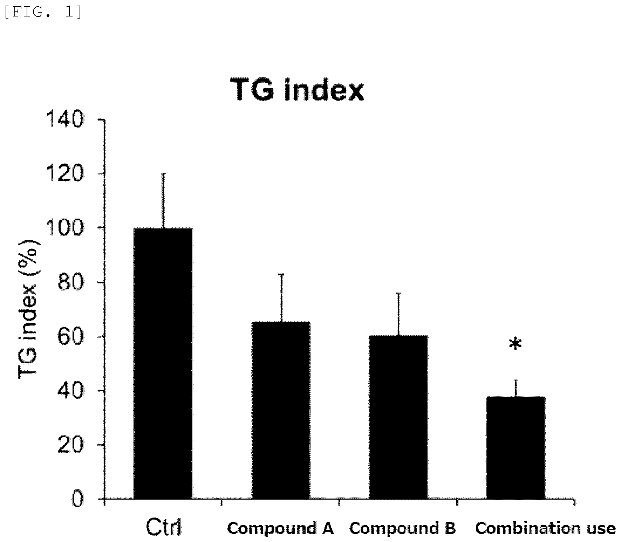

FIG. 1 shows the results of calculated the TG index. Regarding to the TG index, the value of the compound A group was 65.4% and the value of the compound B group was 60.6%. The actual value of the TG index of the combined use group was 37.9% whereas a Burge calculation value was 39.6% (0.654 × 0.606 × 100 = 39.6%). The actual reduction in TG index exceeded that of the theoretical value, that is, a synergistic effect of the compound A and the compound B was observed. Since the combination use group exhibited a significant lowering action as compared with the control group (Dunnet's test), it was revealed that the administration of both the compounds specifically exhibits a lowering action on the TG index (in FIG. 1, the asterisk (*) indicates a significant difference of p < 0.05).

These results indicate that the combination of the compound A and the compound B becomes effective even with a dose with which a significant effect provided by drug treatment is not observed by single administration. That is, it was indicated that the combination of the compound A and the compound B which are the agent, and the pharmaceutical composition of the present invention exhibits a strong action for improving dyslipidemia.

Example 2 Effect of Combination Use of Compound A and Compound B on Expression Level of PPARα Gene in Liver of Rat

### 1. Method

Male rats (7-week-old, Wistar [Crlj: WI], Charles River Laboratories Japan, Inc.) were used for an experiment. Blood was collected from the jugular vein under satiation, and the rats were divided into four groups (N=6) based on TG and TC in the plasma and body weights. For two weeks from the next day of the grouping, each of a solvent (0.5% carboxymethyl cellulose aqueous solution: CMC), a compound A (pemafibrate) alone, a compound B (bempedoic acid) alone, and a combination of pemafibrate and bempedoic acid was orally administered once per day. After four hours from the final administration of the drug, blood was collected under isoflurane anesthesia to obtain the liver. RNA in the liver was extracted using ISOGEN (Nippon Gene Co., Ltd.), and cDNA was prepared using a high-capacity cDNA reverse transcription kit (Thermo Fischer Scientific). Quantitative PCR (polymerase chain reaction) was performed using the prepared cDNA, and the relative value of the expression level of the PPARα gene was calculated using β-actin gene as an internal standard. For PPARα gene detection, Forward primer: 5'-TCATACTCGCAGGAAAGACT-3' (SEQ ID NO: 1) and Reverse primer: 5'-ACCTCTGCCTCCTTGTTTTC-3' (SEQ ID NO: 2) were used. For β-actin gene detection, Forward primer: 5'-AGCCATGTACGTAGCCATCC-3' (SEQ ID NO: 3) and Reverse primer: 5'-ACCCTCATAGATGGGCACAG-3' (SEQ ID NO: 4) were used.

### 2. Group Configuration

| | |
|---|---|
| Group 1: | Control (Solvent Only) |
| Group 2: | 0.3 mg/kg of Compound A |
| Group 3: | 30 mg/kg of Compound B |
| Group 4: | 0.3 mg/kg of Compound A and 30 mg/kg of Compound B |

### 3. Statistical Analysis and Data Processing Method

The results were presented by an average value ± standard error. Comparison between the control group and each of the drug administration groups was performed by a multiple comparison test of Dunnett, and a risk rate of less than 5% was determined to have a significant difference.

### 4. Results

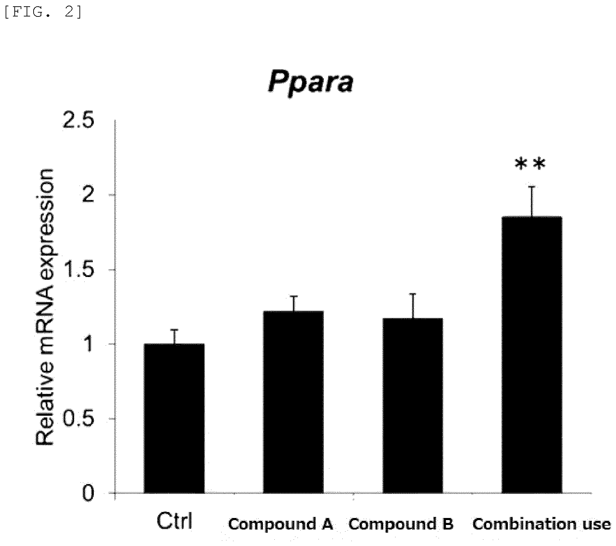

The results showing the expression level of PPARα gene (Ppara) in the liver are shown in FIG. 2. The compound A group and Compound B group showed 1.17 and 1.22 times higher, respectively, than Control. The actual value of the expression level of PPARα gene in the liver of the combined use group was 1.85 times higher than Control, whereas the Burge calculated value was 1.43 times (1.17 × 1.22 = 1.43). The actual increase in the expression level of PPARα gene exceeded that of the theoretical value, that is, a synergistic effect provided by the compound A and the compound B was observed. Since the combination use group exhibited a significant increasing action as compared with the control group (Dunnet's test), it was revealed that the administration of both the compounds specifically exhibits an increasing action on the expression level of PPARα gene in the liver (in FIG. 2, the asterisk (**) indicates a significant difference of p < 0.01).

These results indicate that the combination of the compound A and the compound B becomes effective even with a dose with which a significant effect provided by drug treatment is not observed by single administration. That is, it was indicated that the combination of the compound A and the compound B which are the drug, and the pharmaceutical composition of the present invention exhibits a strong action for improving dyslipidemia.

### Example 3 Effect of Combination Use of Compound A and Compound B on Total Cholesterol Content in Liver of Each of Mice

### 1. Method

Male mice (8-week-old, db/db: BKS.Cg-+ Leprdb/+ Leprdb/Jcl, Lean: BKS.Cg-m +/m +/Jcl, CLEA Japan, Inc.) were used for an experiment. They were divided into four groups (N = 8) based on body weights. For two weeks from the next day of the grouping, each of a solvent (0.5% methylcellulose aqueous solution: MC), a compound A (pemafibrate) alone, a compound B (bempedoic acid) alone, and a combination of pemafibrate and bempedoic acid was orally administered once per day. After four hours from the final administration of the drug, the liver was extracted under isoflurane anesthesia, and the TC concentration in the liver was measured using the FOLCH method described above.

### 2. Group Configuration

| | |
|---|---|
| Group 1: | Control (Solvent Only) |
| Group 2: | 0.3 mg/kg of Compound A |
| Group 3: | 10 mg/kg of Compound B |
| Group 4: | 0.3 mg/kg of Compound A and 10 mg/kg of Compound B |

### 3. Statistical Analysis and Data Processing Method

The results were presented by an average value ± standard error. Comparison between the control group and each of the drug administration groups was performed by a multiple comparison test of Tukey, and a risk rate of less than 5% was determined to have a significant difference.

### 4. Results

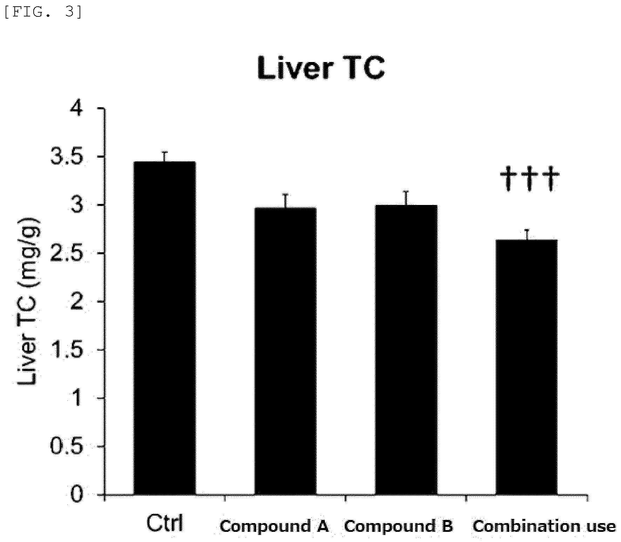

The results showing the total cholesterol content in the liver of each of db/db mice are shown in FIG. 3. With compared to Control, the compound A group was 86.2%, and the compound B group was 87.0%. The total cholesterol content in the liver in the combined use group was 76.5%, and only the combination use group exhibited a significant lowering action as compared with the control group (Tukey's test), and therefore it was revealed that the administration of both the compounds specifically exhibits a lowering action on the total cholesterol content in the liver (in FIG. 3, the cross (+++) indicates a significant difference of p < 0.001).

These results indicate that the combination of the compound A and the compound B becomes effective even with a dose with which a significant effect provided by drug treatment is not observed by single administration. That is, it was indicated that the combination of the compound A and the compound B which are the drug, and the pharmaceutical composition of the present invention exhibits a strong action for improving dyslipidemia.

### Industrial Applicability

The therapeutic agent, medicament, pharmaceutical composition, therapeutic method, use or combination of the present invention have an excellent cholesterol lowering action in the liver and an excellent TG lowering action in the blood, and are useful for prevention and/or treatment for dyslipidemia such as hypercholesterolemia and hypertriglyceridemia, or cardiovascular diseases such as arteriosclerosis and cardiovascular events, and thus have industrial applicability.

## Claims

1. A pharmaceutical composition for preventing and/or treating dyslipidemia or cardiovascular disease, comprising a PPARα agonist and an ACL inhibitor.

2. The pharmaceutical composition according to claim 1, wherein the dyslipidemia is hypercholesterolemia or hypertriglyceridemia.

3. The pharmaceutical composition according to claim 1 or 2, wherein the cardiovascular disease is arteriosclerosis or a cardiovascular event.

4. The pharmaceutical composition according to any of claims 1 to 3, wherein the PPARα agonist is (R)-2-[3-[[N-(benzoxazole-2-yl)-N-3-(4-methoxyphenoxy)propyl]aminomethyl]phenoxy]butyric acid, or a salt, or a solvate, or a solvate of the salt thereof.

5. The pharmaceutical composition according to any of claims 1 to 4, wherein the ACL inhibitor is 8-hydroxy-2,2,14,14-tetramethylpentadecanedioic acid, or a salt, or a solvate, or a solvate of the salt thereof.
